# EUROPÄISCHE PATENTSCHRIFT

(11) **EP 3 596 035 B1**
(45) Veröffentlichungstag und Bekanntmachung des Hinweises auf die Patenterteilung: **26.08.2020**
(21) Anmeldenummer: 19727036.6
(22) Anmeldetag: 03.06.2019
(51) Int. Cl.: C07C 45/58, C07C 49/413

(54) **VERFAHREN ZUR KONTINUIERLICHEN HERSTELLUNG VON KETONEN AUS EPOXIDEN IM FESTBETT**
METHOD FOR THE CONTINUOUS PRODUCTION OF KETONES FROM EPOXIDES IN A FIXED BED
PROCÉDÉ DE PRODUCTION CONTINUE DE CÉTONES À PARTIR DES ÉPOXYDES EN LIT FIXE

(30) Priorität: 07.06.2018 EP 18176493
(43) Veröffentlichungstag der Anmeldung: 22.01.2020
(73) Patentinhaber: Evonik Operations GmbH, 45128 Essen (DE)
(72) Erfinder: MICOINE, Kévin, 45701 Herten (DE); CAMERETTI, Luca, 44267 Dortmund (DE); MEIER, Ralf, 44265 Dortmund (DE); STERGAR, Marcus Matthias, 45699 Herten (DE); SCHULTE-ALTHOFF, Dominik, 45721 Haltern am See (DE); PRINZ, Axel, 63450 Hanau (DE)
(74) Vertreter: Evonik Patent Association
(86) Internationale Anmeldenummer: PCT/EP2019/064319
(87) Internationale Veröffentlichungsnummer: WO 2019/233938

(56) Entgegenhaltungen:
- EP-A1- 2 772 478
- EP-A1- 3 002 058

## Beschreibung

Die vorliegende Erfindung betrifft ein kontinuierliches Verfahren zur Herstellung von Ketonen aus Epoxiden in einer Vorrichtung mit mindestens einem Festbettreaktor.

Verfahren zur Keton-Herstellung aus Epoxiden sind bereits im Stand der Technik bekannt. Hierbei werden häufig kontinuierliche Verfahren zur Ketongewinnung eingesetzt. Die erhaltenen Ketone werden häufig als Ausgangsprodukte für weitere Reaktionen eingesetzt. Beispielsweise wird in EP 2772478 A1 ein Verfahren zur Ketonherstellung aus Epoxiden beschrieben. Dies geschieht in Gegenwart von Metalloxid-Katalysatoren bei Wasserstoffdrücken von 0 bis 0,9 bar.

Bei der kontinuierlichen Herstellung von Ketonen aus Epoxiden entstehen häufig Nebenprodukte, die einen höheren Siedepunkt als die gewünschten Ketone aufweisen. Sie verringern die Ausbeute an Keton und müssen aufwändig abgetrennt werden.

In EP 2743247 A1 (US 2014/0171636 A1) wird ein Verfahren zur Abtrennung von Hochsiedern beschrieben, welches über eine Sequenz mehrerer Seitenabzugskolonnen erfolgen kann. EP 2742981 A2 (US 2014/0166470 A1) schildert ein Abtrennungsverfahren für Hochsieder, wobei eine Kolonne mit einer gelochten Seitenwand eingesetzt wird.

Der Stand der Technik offenbart somit Lösungen, die entstandenen Hochsieder-Nebenprodukte abzutrennen. Nicht erwähnt sind jedoch Verfahren, die Anteile an Hochsiedern während der Reaktion zum Keton signifikant verringern.

Es bestand somit die Aufgabe, ein Verfahren der eingangs genannten Art zur Verfügung zu stellen, welches es erlaubt, den Anteil an Hochsiedern während der Reaktion von Epoxiden zu Ketonen gering zu halten. Hierdurch sollte die Ausbeute an Keton erhöht und der apparative Aufwand zur Abtrennung von Hochsiedern verringert werden.

Überraschend wurde gefunden, dass der Anteil an Hochsiedern niedriggehalten werden kann, wenn der Partialdruck an Kohlenstoffmonoxid gering bleibt. Dabei ist Wasserstoff abwesend oder in geringen Mengen, d.h. bei einem Wasserstoffpartialdruck von maximal 0,2 bar, vorhanden. Demgemäß wird ein Verfahren zur kontinuierlichen Herstellung von Ketonen aus einer mindestens eine Epoxidgruppe enthaltenden Verbindung zur Verfügung gestellt. Das Verfahren wird in einer Vorrichtung umfassend mindestens einen Festbettreaktor, im Folgenden auch kurz Reaktor genannt, durchgeführt, wobei der mindestens eine Festbettreaktor eine Katalysatorzusammensetzung enthält, welche mindestens ein Edelmetall und mindestens ein Metalloxid umfasst. Dabei beträgt der Druck im Reaktor vor dem Katalysator mindestens 1,2 bar. Die Gasphase des Reaktors weist maximal 0,2 bar Wasserstoff-Partialdruck auf; vorzugsweise beträgt der Druck 0 bar, d.h. es ist kein Wasserstoff vorhanden. Im Festbettreaktor wird durch Einleiten mindestens eines Inertgases ein Kohlenstoffmonoxid-Partialdruck in der Gasphase nach dem Katalysator von 50 mbar oder weniger, vorzugsweise 30 mbar oder weniger, eingestellt.

Das Einleiten des Inertgases erfolgt üblicherweise vor dem Festbettreaktor.

In einem kontinuierlichen Verfahren wird Kohlenstoffmonoxid kontinuierlich produziert und ein gewisser Partialdruck an Kohlenstoffmonoxid stellt sich abhängig vom Druck der Anlage und von der Verdünnung durch Inertgase ein. Der Partialdruck an Kohlenstoffmonoxid kann sich im Besonderen in einer industriellen Produktionsanlage auf ein vergleichsweise hohes Niveau einpegeln, weil der Gesamtdruck im Reaktor oberhalb des Katalysators aufgrund großtechnischer Rahmenbedingungen (zum Beispiel Druckverlust über dem Katalysator, Druckniveau in der Abgasleitung) ein Mindestniveau von 1,2 bar beträgt und das Einleiten von Inertgasen (z.B. Stickstoff) aus wirtschaftlichen Gründen begrenzt wird. Obwohl eine Erhöhung des Gesamtdrucks im Reaktor und eine Reduzierung der Einleitung von Inertgasen in einer Produktionsanlage aus erster Sicht wirtschaftlich und technisch vorteilhaft wäre, wurde hier gefunden, dass eine Kontrolle dieser Parameter notwendig ist, um den Partialdruck von Kohlenstoffmonoxid niedrig zu halten.

Die Verringerung des Partialdrucks von Kohlenstoffmonoxid kann durch Verdünnung der Gasphase des Festbettreaktors mit einem Inertgas, ggf. in Verbindung mit einer Verringerung des Gesamtdrucks im Reaktor, erfolgen. Insbesondere kann eine Kontrolle des Partialdrucks an Kohlenstoffmonoxid die Bildung von hochsiedenden Nebenprodukten erheblich reduzieren und damit die Ausbeute an verwertbaren Produkten deutlich erhöhen.

Im Folgenden wird die mindestens eine Epoxidgruppe enthaltende Verbindung als Verbindung E bezeichnet.

Zu den Inertgasen zählen bspw. Stickstoff, Kohlendioxid, Schwefelhexafluorid sowie Edelgase wie Helium und Argon. Bevorzugte Inertgase sind Stickstoff, Kohlendioxid, Argon oder Mischungen davon. Bevorzugte Inertgase sind Stickstoff, Kohlendioxid, Argon oder Mischungen davon, besonders bevorzugt Stickstoff. Vorzugsweise beträgt das Verhältnis der Stoffmenge an Inertgas bzw. Gasgemisch und der Stoffmenge an der Verbindung E mindestens 0,5, bevorzugt 1 und besonders bevorzugt 2.

Eine Verringerung des Partialdrucks an Kohlenstoffmonoxid kann zusätzlich durch Verringerung des Gesamtdrucks im Reaktor erfolgen. Vorzugsweise beträgt der Gesamtdruck im Reaktor oberhalb des Katalysators weniger als 4 bar, besonders bevorzugt weniger als 2 bar, ganz besonders bevorzugt weniger als 1,5 bar.

Die Verbindung E kann aliphatisch oder cycloaliphatisch sein, wobei cycloaliphatische Verbindungen bevorzugt sind. Vorzugsweise sind 4 bis 20 C-Atome, bevorzugt 6 bis 16 C-Atome, besonders bevorzugt 8 bis 14 C-Atome, ganz besonders bevorzugt 10 bis 12 C-Atome und insbesondere 12 C-Atome umfasst.

Die Verbindung E kann eine oder mehrere Epoxid-Gruppen enthalten, wobei Mono-Epoxid-Verbindungen bevorzugt sind.

Weiterhin kann die Verbindung gesättigt oder ungesättigt sein. Beispielsweise können eine oder zwei Doppelbindungen enthalten sein.

Bevorzugte Verbindungen E sind Monoepoxy-Cycloalkane, Monoepoxy-Cycloalkandiene und Monoepoxy-Cycloalkene, wobei Monoepoxy-Cycloalkane besonders bevorzugt sind. Eine ganz besonders bevorzugte Verbindung E ist Monoepoxy-cyclododecan.

Während der kontinuierlichen Umsetzung von Epoxiden zu Ketonen können sich Nebenprodukte bilden. Ein Teil dieser Nebenprodukte haben einen so hohen Siedepunkt, dass sie mittels Gaschromatographie nicht erfasst werden. Die Bildung solcher Nebenprodukte stellt einen Selektivitätsverlust dar. Um alle möglichen Verunreinigungen bei der Berechnung der Selektivität zu berücksichtigen, wurden die Reaktionsgemische mittels Gaschromatographie (GC) unter Zugabe einer bekannten Menge Tetradecan als externer Standard analysiert. Für alle bekannten Stoffe, die bei der Reaktion bspw. von Epoxycyclododecan vorliegen (Tetradecan, Cycloundecan, Cycloundecen, Cyclododecan, Cyclododecen, Cyclododecanon, Cyclododecanol, Epoxycyclododecan), können GC-Faktoren mit angesetzten Referenzlösungen ermittelt werden. Folglich kann durch Zugabe einer bekannten Menge Tetradecan in jede Reaktionsprobe für jeden Stoff ein massenbezogenen Anteil in jeder Reaktionsmischung berechnet werden. Aus dem GC-Chromatogramm können die Anteile aller Stoffe berechnet werden, die im Chromatogramm sichtbar sind. Der Anteil der hochsiedenden Nebenprodukte (Hochsieder), die im GC nicht erfasst werden, kann durch die Differenz zwischen 100 % und der Summe aller Stoffe im GC Chromatogramm berechnet werden.

Zu den Hochsiedern zählen diejenigen Reaktionsprodukte aus der mindestens eine Epoxidgruppe enthaltenden Verbindung, die gegenüber dem entstandenen Keton einen um mindestens 10 K höheren Siedepunkt bei 1013 hPa aufweisen. Sie lassen sich durch weitere Verfahren nicht in das gewünschte Keton umsetzen (nicht-verwertbare Produkte). Zu den Hochsiedern zählen im Falle von Epoxycyclododecan beispielsweise Cyclododecan-1-on-2-ol, Cyclododecan-1,2-diol, C12-Dimere (also Verbindungen mit 24 C-Atomen), C12-Trimere (also Verbindungen mit 36 C-Atomen) und weitere Oligomere. Im Gegensatz dazu sind erhaltene Nebenprodukte wie das Alkan, das Alken, das Alkenon, der Alkohol und das Alkenol verwertbare Produkte, die sich durch bekannte Verfahren des Standes der Technik in das gewünschte Keton umsetzen lassen (verwertbare Produkte), und stellen somit keinen Selektivitätsverlust dar. Im Falle von Epoxycyclododecan zählen hierzu Cyclododecan (CDAN), Cyclododecen (CDEN), Cyclododecanon (CDON), Cyclododecenon (CDENON), Cyclododecanol (CDOL) und Cyclododecenol (CDENOL).

Da die hochsiedenden Nebenprodukte einen unverwertbaren Abfall darstellen, sollte derer Anteil so gering wie möglich sein. Vorzugsweise beträgt dieser Anteil weniger als 5 Gew.-% bezogen auf das umgesetzte Epoxid, besonders bevorzugt weniger als 2 Gew.-%.

Es wurde überraschend gefunden, dass Kohlenstoffmonoxid als Nebenprodukt während der Umsetzung von Epoxiden zu Ketonen gebildet wird und im kontinuierlichen Verfahren einen signifikanten Anteil der Gasphase des Reaktors darstellt. Die Bildung von Kohlenstoffmonoxid lässt sich beispielsweise im Falle von Epoxycyclododecan durch die Nebenreaktion zu Cycloundecan und Cycloundecen erklären. Es können sich 1 bis 2 mol-% Cycloundecan und Cycloundecen bezogen auf Epoxycyclododecan während der Reaktion bilden, was einer Bildung von 1 bis 2 mol-% CO entspricht. Abhängig von der dosierten Gasmenge in der Anlage kann der Kohlenstoffmonoxidanteil folglich mehrere Prozente der Gasphase entsprechen.

Der Partialdruck von Kohlenstoffmonoxid wird aus dem Anteil an Kohlenstoffmonoxid in der Gasphase und dem Gesamtdruck berechnet. Der Anteil an Kohlenstoffmonoxid kann beispielsweise durch FT-IR-Spektrometrie online mit einer Sonde ermittelt werden. Es wurde hierbei gefunden, dass dieser Partialdruck in der Abwesenheit von oder bei geringen Partialdrücken von bis zu 0,2 bar an Wasserstoff einen erheblichen Einfluss auf die Selektivität der chemischen Reaktion hat. Ein höherer Partialdruck an Kohlenstoffmonoxid führt zu einer Verringerung der Keton-Selektivität und einer Erhöhung des Anteils an Hochsiedern. Dieser Effekt ist reversibel und die Keton-Selektivität wird besser, sobald der CO-Partialdruck wieder reduziert wird. Eine mögliche Erklärung ist eine Vergiftung des edelmetallhaltigen Katalysators durch Kohlenstoffmonoxid, die den gewünschten Reaktionsweg zum Keton hemmt.

Wenn genug Wasserstoff in den Reaktor eingeleitet wird, wird diese Vergiftung des edelmetallhaltigen Katalysators nicht beobachtet: Der Anteil an Hochsiedern wird nicht erhöht, auch bei höheren Konzentrationen an Kohlenstoffmonoxid. Allerdings möchte man im industriellen Verfahren auf das Einleiten von Wasserstoff verzichten, weil es zu einer geringeren Selektivität zum Keton und zu einer erhöhten Selektivität zu den Nebenprodukten Alkohol und Alkan führt. Aus diesen Gründen wird das erfindungsgemäße Verfahren auf einen Wasserstoffpartialdruck von maximal 0,2 bar limitiert.

Die Temperatur während der Reaktion wird vorzugsweise auf einen Bereich von 100 bis 350 °C, bevorzugt 150 bis 250 °C und besonders bevorzugt zwischen 180 und 230 °C eingestellt. Die Reaktion kann durchgeführt werden mit einer Verbindung E, die sich im flüssigen oder gasförmigen Zustand befindet.

Die kontinuierliche Umlagerung von Epoxiden zu Ketonen in einem Festbettreaktor erfolgt vorzugsweise in Gegenwart eines edelmetallhaltigen Katalysators (Katalysatorsystem), wobei der Katalysator bevorzugt Titandioxid, Zirconiumdioxid oder beides enthält.

Das Edelmetall des Katalysatorsystems wird vorzugsweise ausgewählt aus Ruthenium, Rhodium, Palladium, Osmium, Iridium und Platin, wobei Ruthenium, Palladium und Platin bevorzugt sind und Palladium besonders bevorzugt ist. Das Edelmetall kann als Pulver (ungeträgert) oder geträgert vorliegen. In Pulverform sind beispielsweise elementare Edelmetalle oder ihre Oxide geeignet.

Weiterhin kann mindestens ein Metalloxid als weiterer Bestandteil des Katalysatorsystems enthalten sein. Das Metalloxid des Katalysatorsystems umfasst Titandioxid, Zirconiumdioxid oder Mischungen daraus oder besteht aus mindestens einem der zuvor genannten Oxide. Beispielweise kann das Metalloxid des Katalysatorsystems ein Mischoxid umfassen, wobei das Mischoxid Zirconiumdioxid und Siliciumdioxid enthält.

Das Metalloxid des Katalysatorsystems kann als Träger für das Edelmetall des Katalysatorsystems fungieren. Das Edelmetall kann wahlweise auf einem alternativen Träger aufgebracht sein, der beispielsweise aus Aluminiumoxid, Siliciumdioxid oder Aktivkohle ausgewählt ist. Titandioxid, Zirconiumdioxid oder Mischoxide enthaltend Zirconiumdioxid sind bevorzugte Träger.

Die Metalloxide des Katalysatorsystems sowie die alternativen Träger können als Pulver oder als Formkörper vorliegen. Geeignete Formkörper sind Kugeln, Extrudate, Tabletten, Granulate und Pellets. Es ist bevorzugt, dass die Träger des Edelmetalls als Formkörper vorliegen. Es ist ebenso bevorzugt, dass das Metalloxid des Katalysatorsystems, wenn es nicht als Träger fungiert, als Formkörper vorliegt.

Das Katalysatorsystem kann unabhängig voneinander als eines der folgenden Systemformen vorliegen:
I) Das Edelmetall ist auf einem Metalloxid geträgert, welches aus Titandioxid und Zirconiumdioxid ausgewählt ist, wobei vorzugsweise kein Titandioxid enthalten ist.
II) Das Edelmetall ist geträgert, wobei der Träger nicht Titandioxid und/oder Zirconiumdioxid enthält oder daraus besteht. Das System enthält zusätzlich zumindest ein Metalloxid, welches ausgewählt ist aus Titandioxid oder Zirconiumdioxid.

Geeignetes Titandioxid als Metalloxid des Katalysatorsystems kann durch das Sulfatverfahren, das Chloridverfahren oder durch Flammhydrolyse (Pyrogenverfahren) von Titantetrachlorid erhalten werden. Sämtliche Verfahren sind dem Fachmann bekannt. Geeignete Modifikationen sind Rutil und Anatas, wobei das eingesetzte Titandioxid Mischungen der genannten Modifikationen enthalten kann.

Besonders bevorzugtes Titandioxid wird mittels Flammpyrolyse gewonnen, wie es beispielsweise in DE-C-830786 beschrieben ist.

Geeignetes Titandioxid ist unter der Bezeichnung Aeroxid P25 Titandioxid (Pulver) oder Aerolyst 7711 (Formkörper) der Firma Evonik, Deutschland, sowie Hombikat M234 (Formkörper) der Firma Sachtleben, Deutschland, erhältlich.

Zirconiumdioxid (Zirconium(IV)-oxid) ist beispielsweise aus Zirconiumhydroxid erhältlich, wobei es bei über 200 °C, beispielsweise bei 350 °C, kalziniert wird.

Besonders geeignete Mischoxide als Metalloxid umfassen Zirconiumdioxid und Siliciumdioxid oder bestehen aus diesen beiden Oxiden. Der Anteil der Summe aus Zirconiumdioxid und Siliciumdioxid im Mischoxid beträgt vorzugsweise mindestens 20 Gew.-%, und bevorzugt mindestens 30 Gew.-%, besonders bevorzugt 50 Gew.-% und ganz besonders bevorzugt 95 Gew.-%, jeweils bezogen auf das Gesamtgewicht des Mischoxids. Besonders bevorzugt besteht das Mischoxid aus Zirconiumdioxid und Siliciumdioxid. Bevorzugt beträgt das Massenverhältnis von Zirconiumdioxid zu Siliciumdioxid im Mischoxid 86 : 14 bis 99,9 : 0,1.

Mischoxide können aus der Calcination einer Zirconiumverbindung mit einem Siliciumdioxid entstehen. Vorzugsweise wird das Siliciumdioxid mittels Pyrogenverfahren hergestellt. Das Verfahren ist dem Fachmann bekannt, z.B. aus der Schriftenreihe "Fine Particles" Nr. 11 (7. Ausgabe, 2003), Firmenzeitschrift der Degussa AG. Die Zirconiumverbindung ist vorzugsweise ausgewählt aus Zirconiumdioxid, Zirconiumhydroxid, Zirconiumacetat, Zirconiumnitrat, Zirconiumoxychlorid, Ammoniumzirconiumcarbonat oder Mischungen daraus. Bevorzugt werden Zirconiumdioxid, Zirconiumhydroxid oder Mischungen daraus eingesetzt. Als Zirconiumhydroxid wird Zirconium-(IV)-hydroxid verstanden.

Das Metalloxid des Katalysatorsystems kann eine durchschnittliche Schüttdichte von 0,5 bis 2 g/cm³ aufweisen. Die Schüttdichte wird gemessen, indem zunächst ein leerer 1000-mL-Meßzylinder gewogen wird. Anschließend wird das Metalloxid bis zur 500-mL-Marke eingefüllt. Der gefüllte Zylinder wird erneut gewogen, aus der Gewichtsdifferenz von gefülltem und leerem Messzylinder wird die Schüttdichte des Materials in g/cm³ angegeben.

Das Metalloxid des Katalysatorsystems weist vorzugsweise eine BET-Oberfläche auf, die im Bereich von 5 - 155 m²/g liegt.

Es ist bevorzugt, dass die BET-Oberfläche des Metalloxids der erfindungsgemäßen Katalysatorsysteme in einem Bereich von 80 bis 150 m²/g liegt. Die BET-Oberfläche wird gemäß DIN 66131 und DIN ISO 9277 gemessen. Eine BET-Oberfläche oberhalb von 155 m²/g führt zu einer geringeren Selektivität.

Der Anteil an Edelmetall, bezogen auf das Gesamtgewicht aus Edelmetall und Träger, kann 0,01 bis 5 Gew.-%, vorzugsweise 0,05 bis 1,2 Gew.-% und bevorzugt 0,1 bis 0,6 Gew.-% betragen.

Das Edelmetall kann auf oder im Träger verteilt sein.

Der Stoffmengenanteil an Edelmetall, bezogen auf die Stoffmenge der Verbindung E, kann 0,00001 bis 0,1, vorzugsweise 0,0001 bis 0,01, betragen.

Der Stoffmengenanteil an Metalloxid des Katalysatorsystems, bezogen auf die Stoffmenge der Verbindung E, kann 0,01 bis 100, vorzugsweise 0,01 bis 10, betragen.

Zur Herstellung des Systems II kann das Edelmetall auf einen inerten Träger imprägniert werden, wobei der Träger nicht aus dem Metalloxid besteht. Vorzugsweise enthält der Träger nicht das Metalloxid oder besteht daraus. Zu diesem Zweck kann jedes dem Fachmann bekannte Imprägnierungsverfahren wie der Auftrag einer Edelmetalllösung auf den Träger eingesetzt werden.

Zur Herstellung des Systems I kann das Edelmetall auf dem Metalloxid als Träger imprägniert werden. Zu diesem Zweck kann jedes dem Fachmann bekannte Imprägnierungsverfahren wie der Auftrag einer Edelmetalllösung auf den Träger eingesetzt werden.

Das erfindungsgemäße Verfahren kann bei einem Wasserstoffpartialdruck von 0 bis zu 0,2 bar, vorzugsweise 0 bis zu 0,1 bar, ausgeführt werden. Bevorzugt wird das erfindungsgemäße Verfahren ohne Wasserstoff durchgeführt.

Die zuvor genannten Druckangaben beziehen sich auf den Partialdruck an Wasserstoff in dem System. Üblicherweise sind Komponenten des Reaktionsgemisches, einschließlich des Lösemittels, Luft oder Inertgase weitere gasförmige Bestandteile des Systems.

Das Verfahren wird in mindestens einem Festbettreaktor durchgeführt. Alternativ ist dem Festbettreaktor mindestens ein weiterer Festbettreaktor nachgeschaltet. Das Abgas der Festbettreaktoren (einschließlich des ersten Festbettreaktors) kann entweder in einen weiteren Reaktor geführt oder aus dem System entfernt werden.

Falls das Verfahren mehrere nacheinander geschaltete Reaktoren in Serie enthält, kann frisches Gas in jeden Reaktor eingeleitet werden (sogenannte Kreuzsstromfahrweise). Alternativ kann frisches Gas in einen Reaktor eingeleitet werden, dessen Abgas wiederum in den nächsten Reaktor eingeleitet wird. Dadurch wird das Kohlenstoffmonoxid in jedem Reaktor verdünnt (sogenannte Gleichstromfahrweise). Vorzugsweise wird die Einleitung von Gas im Gegenstrom geführt: Frisches Gas wird in den letzten Reaktor eingeleitet und der Gasstrom wird anschließend in den voranstehenden Reaktor geführt, und so weiter bis zum ersten Reaktor, dessen Abgas aus dem Verfahren ausgetragen wird. Dadurch kann die benötigte gesamte Menge an frischem Gas reduziert werden. Auch eine Kombination an Kreuz-, Gleich- und Gegenstromfahrweise kann alternativ gewählt werden.

Der Festbettreaktor ist zumindest zum Teil mit dem edelmetallhaltigen Katalysatorsystem gefüllt. Der darin befindliche Katalysator wird auch als Katalysatorbett bezeichnet. Der Reaktor kann beispielweise als Kreislaufreaktor oder als Rohrreaktor ("plug-flow") betrieben werden. Das Reaktionsgemisch (flüssige Phase und Gasphase) wird durch den Reaktor beispielweise mit einer Pumpe geleitet und damit mit dem Katalysator in Kontakt gebracht. Nach dem Reaktor werden die flüssige Phase und die Gasphase getrennt und mindestens ein Teil der Gasphase wird durch eine Abgasleitung aus der Anlage abgeführt. Ein gewisser Masse-Feed enthaltend das Epoxid wird kontinuierlich vor dem Reaktor oder in den Kreislauf dosiert und die gleiche Masse Reaktionsgemisch wird nach dem Reaktor oder aus dem Kreislauf abgeführt, sodass die Masse des Reaktionsgemisches in der Anlage konstant bleibt. Außerdem kann ein oder mehrere Gase wie beispielweise Stickstoff, Argon und Wasserstoff in die Anlage dosiert werden. Vorzugsweise erfolgt diese Dosierung vor dem Festbettreaktor. Der Druck im Reaktor kann beispielweise durch einen Vordruckregler im Abgasweg eingestellt werden.

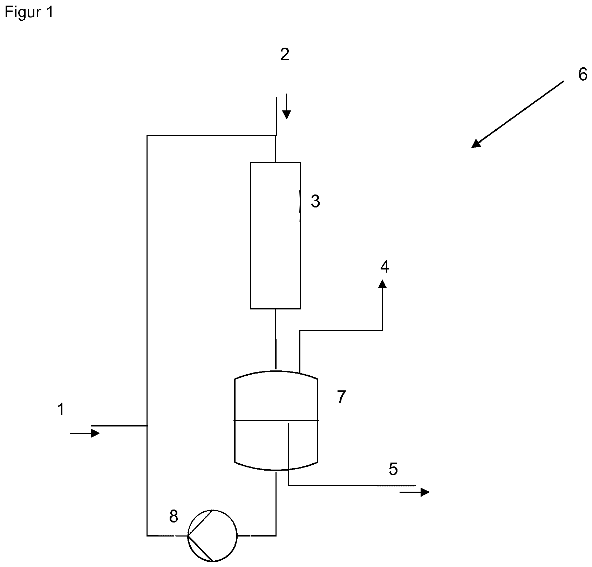

Figur 1 zeigt eine Vorrichtung (6) mit einem Kreislaufreaktor, d.h. einen Festbettreaktor (3), der im Kreislauf betrieben wird. Dort wird der Feed (1) in das System eingeführt. Inertgas (2) wird vor dem oder den Festbettreaktor(en) (3) enthaltend das Katalysatorsystem eindosiert. Abgas (4) und das flüssige Produktgemisch (5) werden in einem Abscheider (7) abgetrennt. Das Abgas wird aus dem System entfernt. Das Produktgemisch wird zum Teil zurück zu dem oder den Festbettreator(en) geführt und zum Teil zum nächsten Verfahrensschritt geführt. Der Druck in der Vorrichtung (6) wird über eine Pumpe (8) erhöht. Üblicherweise ist der Druck zwischen Pumpe und Katalysator, der sich im Festbettreaktor (3) befindet, gegenüber anderen Bereichen der Vorrichtung erhöht. Dies entspricht dem zuvor genannten Druck im Reaktor vor dem Katalysator, welcher mindestens 1,2 bar beträgt.

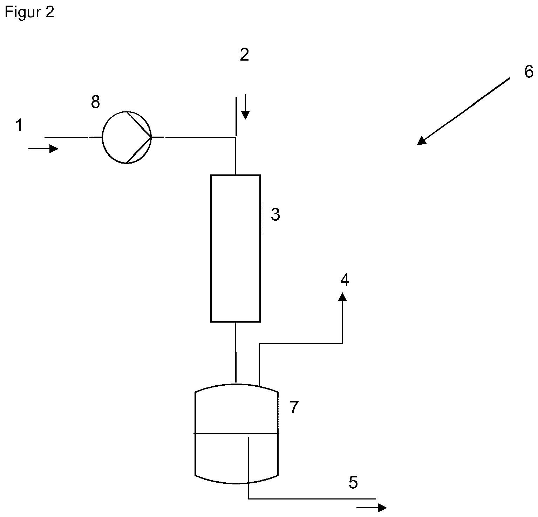

In Figur 2 wird eine Vorrichtung (6) mit Pumpe (8), einem Rohrreaktor als Festbettreaktor (3) dargestellt, in dem der Feed (1) aufgegeben und Inertgas (2) vor dem Festbettreaktor (3) zugegeben wird. Abgas (4) und Produkt (5) werden im Abscheider (7) abgetrennt.

Das erfindungsgemäße Verfahren kann in organischen Lösemitteln durchgeführt werden, wobei es bevorzugt ist, ohne Lösemittel zu arbeiten und somit keine organischen Lösemittel einzusetzen. Geeignete Lösemittel sind beispielsweise Alkane wie n-Hexan, n-Heptan, n-Tetradecan und Cyclohexan; Ether wie Tetrahydrofuran und Dioxan; Alkanole wie Methanol, Ethanol und t-Butanol; Ester wie Ethylacetat und Butylacetat. Die Lösemittel können für sich allein oder in Mischungen eingesetzt werden. Das Lösemittel wird vorzugsweise in einer Menge eingesetzt, welche das 20-fache oder weniger, bevorzugt das 10-fache oder weniger des Gewichts der Verbindung E, beträgt.

In einer bevorzugten Ausführungsform der Erfindung wird Monoepoxy-cyclododecan zu Cyclododecanon ohne Lösemittel bei Temperaturen von 170 bis 250 °C mit einem Festbettkatalysator kontinuierlich umgesetzt, wobei der Partialdruck an Kohlenstoffmonoxid in der Gasphase des Reaktors nach dem Katalysator unter 50 mbar kontrolliert wird. Der Partialdruck an Kohlenstoffmonoxid wird beispielweise durch Messung des Gesamtdrucks mittels Manometer und des Kohlenstoffmonoxidanteils mittels FT-IR Spektrometrie bestimmt.

Ein weiterer Gegenstand der Erfindung ist ein Verfahren zur Synthese von Lactamen (erfindungsgemäßes Lactamverfahren), bei dem das zuvor genannte erfindungsgemäße Verfahren zur Herstellung von Ketonen herangezogen wird: Zunächst erfolgt eine Umlagerung einer mindestens eine Epoxidgruppe enthaltenden Verbindung (Verbindung E) zum Keton. Anschließend wird eine Oximierung des Ketons zum Oxim durchgeführt. Danach erfolgt eine Beckmann-Umlagerung des Oxims zum Lactam. Die Verbindung E wird vorzugsweise ausgewählt aus aliphatischen Monoepoxy-Cycloalkane, aliphatischen Monoepoxy-Cycloalkandienen und aliphatischen Monoepoxy-Cycloalkenen, wobei Monoepoxy-Cycloalkane bevorzugt sind.

Sofern das Keton in einem Gemisch mit dem entsprechenden Alkohol-Derivat vorliegt, kann eine Dehydrierung des Alkohols zum Keton erfolgen. Die Beckmann-Umlagerung kann mittels Schwefelsäure oder Cyanurchlorid erfolgen. Die Lactame können unter Polykondensation zu Polyamiden weiterverarbeitet werden.

Die Dehydrierung, die Oximierung, die Beckmann-Umlagerung sowie die Kondensationsreaktion sind dem Fachmann bekannt.

In einer bevorzugten Ausführungsform des erfindungsgsgemäßen Lactamverfahrens wird aus Monoepoxy-cyclododecan (bzw. Cyclododecanepoxid oder 1,2-Cyclododecanepoxid) Laurinlactam hergestellt.

Im Rahmen des bevorzugten Lactamverfahrens kann Monoepoxy-cyclododecan gewonnen werden durch folgende Reaktionsschritte: 1,3-Butadien wird durch Cyclotrimerisation zu Cyclododecatrien umgesetzt. Anschließend erfolgt eine Hydrierung zum Cyclododecen. Durch nachfolgende Epoxidierung wird das Cyclododecanepoxid erhalten. Der Fachmann auf dem Gebiet der Synthese organischer Verbindungen kann andere aliphatische und cycloaliphatische Verbindungen E in Analogie zu der Synthese von Monoepoxy-cyclododecan herstellen.

Nachfolgend wird die vorliegende Erfindung anhand von Beispielen näher erläutert. Alternative Ausführungsformen der vorliegenden Erfindung sind in analoger Weise erhältlich.

### Bezugszeichenliste

- 1: Feed
- 2: Inertgas
- 3: Festbettreaktor
- 4: Abgas
- 5: Produkt
- 6: Vorrichtung
- 7: Abscheider
- 8: Pumpe

### Beispiele

Die prozentuale Angabe bei Katalysatoren gibt den Gewichtsanteil des Edelmetalls an bezogen auf das Gesamtgewicht des Katalysators umfassend Edelmetall und Träger. Die Abkürzung "calc." steht für "calciniert". Die Abkürzungen der Stoffe sind: CDAN: Cyclododecan; CDEN: Cyclododecen; ECD: Epoxycyclododecan; CDON: Cyclododecanon; CDENON: Cyclododecenon (Isomerenmischung); CDOL: Cyclododecanol; CDENOL: Cyclododecenol (Isomerenmischung).

Das eingesetzte Katalysatorsystem bestand aus einem ZrO₂-SiO₂ Mischoxid (95 % ZrO₂, 5 % SiO₂) und einem 0,5 % Pd/SiO₂ Katalysator. Beide Katalysatoren wurden gemäß EP3006107 hergestellt (Mischoxid entsprechend Beispiel B und Pd/SiO₂ entsprechend Beispiel D).

Gaschromatographie (GC): Gaschromatographische Untersuchungen erfolgten mit einem GC-2010 (Shimadzu) Chromatographen, ausgestattet mit Autosampler, Flammenionisationsdetektor (FID) sowie GC-Kapillarsäule Supelcowax® (60 m x 0.32 mm x 0.25 µm, Supelco). Messungen wurden im Split-Modus (Split-Rate 1 : 66) mit Helium als Trägergas (Flussrate 0,89 mL/min, lineare Trägergasgeschwindigkeit 17,9 cm/s) durchgeführt. Temperaturprogramm für GC-Ofen: Starttemperatur 150 °C; mit 5 °C/min bis auf 180 °C heizen, für 10 min halten; mit 5 °C/min bis auf 200 °C heizen, für 10 min halten. Detektor- und Injektortemperaturen betrugen 340 °C und 220 °C.

Durch Zugabe eines externen Standards (Tetradecan) in jede Probe und Anwendung des Faktorverfahrens wurde die Zusammensetzung des Reaktionsgemisches in Gew.-% berechnet. Mit der Molmasse jedes Stoffes konnte dann die Zusammensetzung des Gemisches in mol-% berechnet werden. Man konnte anschließend den Umsatz des Epoxids berechnen. Die Selektivität jedes Produkts wurde anhand der Konzentrationsdifferenz dieses Produkts im Reaktionsgemisch und im Edukt, bezogen auf das umgesetzte Epoxid, berechnet. Für die Hochsieder wurde die Selektivität anhand der Molmasse des Epoxids berechnet, was eine Aussage über die Stoffmenge des Epoxids gibt, die zu Hochsiedern umgesetzt wurde (Selektivitätsverlust während der Reaktion).

Der Anteil an Kohlenstoffmonoxid wurde mit IR-Spektrometrie durch Einleiten des Abgases aus dem Reaktor in ein IR Spektrometer ermittelt. Die spektroskopischen Messungen wurden mit einem Fouriertransform (FT) mid-IR Spektrometer Gasmet DX4000 der Firma Ansyco durchgeführt, das die Absorption im Spektralbereich von 600-4200 cm⁻¹ auflöst. Der gasförmige Stoffstrom aus dem Reaktor wurde mit entsprechenden Leitungen zur Messzelle des Spektrometers geführt und mittels Swagelok-Anschlüssen befestigt. Zur Vermeidung von Kondensation im Stoffstrom wurden die Leitungen mittels elektrischer Begleitheizung auf 110 °C temperiert. In dem verwendeten mid-IR Spektrometer diente ein keramisches SiC-Material (Globar) als mid-IR Quelle. Die Messzelle verfügte über ein Volumen von 0.45 L und einer optischen Weglänge von 500 cm, welche über Mehrfachreflexionen realisiert wurde. Die Messzelle wurde ebenfalls auf eine Temperatur von 110 °C temperiert. Als Detektor diente ein thermoelektrisch gekühlter MCT-Detektor. Die Kalibrierung der CO-Bestimmung erfolgte anhand von Referenzspektren ausgehend von Prüfgasen (Bereich: 0.9 bis 7 Vol% Kohlenstoffmonoxid in Stickstoff). Hierfür wurde der spektrale Bereich des CO-Signals von 1850-2060 cm⁻¹ ausgewertet, da hier keine Interferenzen mit weiteren Komponenten beobachtet wurden. Die Kalibrierung und die Messungen wurden mit der Software Calcmet der Firma Ansyco durchgeführt. Die Messungen der Gasphase des Reaktors wurden gegen reinen Stickstoff als Hintergrundspektrum durchgeführt. Als Messdauer wurden 60 s bei einem Messintervall von 1800 s verwendet.

Die Angaben zum Massenstrom des Stickstoffs werden zur Unterscheidung zum Volumenstrom in NL/h angegeben. Als Norm wird DIN 1343 herangezogen (Gegendruck 1013,25 mbar, Gastemperatur 0 °C).

### Beispiel 1: nicht erfindungsgemäß

Die Reaktion wurde in einer Laboranlage durchgeführt. Die Anlage bestand aus zwei Festbettreaktoren in Serie (ca. 200 mL pro Reaktor) und einem Vorlagebehälter (1 L). Der untere Festbettreaktor wurde mit 45 g ZrO₂-SiO₂ Mischoxid (95% ZrO₂, 5% SiO₂) und der obere Festbettreaktor wurde mit 90 g von 0,5% Pd/SiO₂ befüllt. Der Behälter wurde mit 1000 g Cyclododecanon befüllt. Die Flüssigkeit wurde im Kreis aus der Vorlage über das Katalysatorbett zurück in den Vorlagebehälter mittels einer Umwälzpumpe (10 I/h) gepumpt. Die Reaktoren wurden bis zu einer internen Temperatur von 185°C im Reaktionsgemisch mit einem Thermostat aufgeheizt.

Dann wurden kontinuierlich 65 g/h Feed enthaltend 85,4 Gew% Epoxycyclododecan, 9,3 Gew% CDAN und 5,3% CDEN in den Kreislauf dosiert. Dies entspricht einer Dosierung von 0,31 mol/h Epoxycyclododecan. Aus der Anlage wurde kontinuierlich Produkt über ein Überlaufrohr ausgetragen, so dass der Füllstand im Vorlagebehälter konstant blieb. Außerdem wurden kontinuierlich 2 NL/h Stickstoff in die Anlage dosiert, was einer Stoffmenge von 0,08 mol/h N₂ entspricht. Somit betrug das Verhältnis der Stoffmenge Stickstoff zu der Stoffmenge Epoxycyclododecan 0,26. Mit einem Vordruckregler wurde ein Gesamtdruck in der Anlage von 3,2 bar eingestellt und das Abgas wurde kontinuierlich aus der Anlage ausgetragen.

Nach 48 h Laufzeit war das Reaktionsgemisch im stationären Zustand. Es wurde ein Umsatz des Epoxids von 77% erreicht. Die Selektivität zum CDON betrug lediglich 6,8 mol-% und es wurden 17 mol-% Hochsieder gebildet.

Der Anteil von Kohlenstoffmonoxid im Abgas wurde mit IR-Spektrometrie ermittelt und betrug 2,6 mol-%. Bei einem Gesamtdruck von 3,2 bar wurde ein Partialdruck von Kohlenstoffmonoxid von 83 mbar erreicht.

**Tabelle 1. Umsatz des Epoxids (mol-%, GC mit externem Standard) und Selektivität der verschiedenen Produkte mol-%, GC mit externem Standard)**

| **Umsatz Epoxid (mol%)** | **Selektivität (mol%)** | | | | | | | |
|---|---|---|---|---|---|---|---|---|
| | **Cycloundecan + Cycloundecen** | **CDAN** | **CDEN** | **CDON** | **CDENON** | **CDOL** | **CDENOL** | **Hochsieder** |
| 77 | 0,2 | 0,1 | 6,5 | 6,8 | 8,3 | 1,0 | 60,1 | 17 |

CDAN, CDEN, CDON, CDENON, CDOL und CDENOL sind Stoffe, die alle mit bekannten Verfahren zu CDON umgesetzt werden können. Sie sind also alle verwertbaren Produkte. Aus diesem Grund beträgt die Selektivität verwertbarer Produkte 82,8 mol%.

### Beispiel 2: nicht erfindungsgemäß

Die Reaktion wurde in einer Laboranlage gemäß Beispiel 1 durchgeführt. Es wurden kontinuierlich 65 g/h Feed enthaltend 85,4 Gew% Epoxycyclododecan, 9,3 Gew% CDAN und 5,3 % CDEN in den Kreislauf dosiert. Dies entspricht einer Dosierung von 0,31 mol/h Epoxycyclododecan. Aus der Anlage wurde kontinuierlich Produkt über ein Überlaufrohr ausgetragen, so dass der Füllstand im Vorlagebehälter konstant blieb. Außerdem wurden kontinuierlich 5 NL/h Stickstoff in die Anlage dosiert, was einer Stoffmenge von 0,20 mol/h N₂ entspricht. Somit betrug das Verhältnis der Stoffmenge Stickstoff zu der Stoffmenge Epoxycyclododecan 0,67. Mit einem Vordruckregler wurde ein Gesamtdruck in der Anlage von 3,2 bar eingestellt und das Abgas wurde kontinuierlich aus der Anlage ausgetragen.

Nach 48 h Laufzeit war das Reaktionsgemisch im stationären Zustand. Es wurde ein Umsatz des Epoxids von 78 % erreicht. Die Selektivität des CDON betrug 37 mol-% und es wurden 16,5 mol-% Hochsieder gebildet.

Der Anteil von Kohlenstoffmonoxid im Abgas wurde mit IR-Spektrometrie ermittelt und betrug 1,83 mol-%. Bei einem Gesamtdruck von 3,2 bar wurde ein Partialdruck von Kohlenstoffmonoxid von 59 mbar erreicht.

**Tabelle 2. Umsatz des Epoxids (mol-%, GC mit externem Standard) und Selektivität der verschiedenen Produkte (mol-%, GC mit externem Standard)**

| **Umsatz Epoxid (mol-%)** | **Selektivität (mol-%)** | | | | | | | |
|---|---|---|---|---|---|---|---|---|
| | **Cycloundecan + Cycloundecen** | **CDAN** | **CDEN** | **CDON** | **CDENON** | **CDOL** | **CDENOL** | **Hochsieder** |
| 78 | 1,0 | 0,1 | 2,0 | 37,4 | 11,3 | 3,4 | 28,3 | 16,5 |

Die Selektivität verwertbarer Produkte betrug 82,5 mol-%.

### Beispiel 3: erfindungsgemäß

Die Reaktion wurde in einer Laboranlage gemäß Beispiel 1 durchgeführt. Es wurden kontinuierlich 65 g/h Feed enthaltend 85,4 Gew% Epoxycyclododecan, 9,3 Gew% CDAN und 5,3 % CDEN in den Kreislauf dosiert. Dies entspricht einer Dosierung von 0,31 mol/h Epoxycyclododecan. Aus der Anlage wurde kontinuierlich Produkt über ein Überlaufrohr ausgetragen, so dass der Füllstand im Vorlagebehälter konstant blieb. Außerdem wurden kontinuierlich 20 NL/h Stickstoff in die Anlage dosiert, was eine Stoffmenge von 0,81 mol/h N₂ entspricht. Somit betrug das Verhältnis der Stoffmenge Stickstoff zu der Stoffmenge Epoxycyclododecan 2,6. Mit einem Vordruckregler wurde ein Gesamtdruck in der Anlage von 3,2 bar eingestellt und das Abgas wurde kontinuierlich aus der Anlage ausgetragen.

Nach 48 h Laufzeit war das Reaktionsgemisch im stationären Zustand. Es wurde ein Umsatz des Epoxids von 79 % erreicht. Die Selektivität des CDON betrug 90 mol-% und es wurden lediglich 2 mol-% Hochsieder gebildet.

Der Anteil von Kohlenstoffmonoxid im Abgas wurde mit IR-Spektrometrie ermittelt und betrug 0,85 mol-%. Bei einem Gesamtdruck von 3,2 bar wurde ein Partialdruck von Kohlenstoffmonoxid von 27 mbar erreicht.

**Tabelle 3. Umsatz des Epoxids (mol-%, GC mit externem Standard) und Selektivität der verschiedenen Produkte (mol-%, GC mit externem Standard)**

| **Umsatz Epoxid (mol-%)** | **Selektivität (mol-%)** | | | | | | | |
|---|---|---|---|---|---|---|---|---|
| | **Cycloundecan + Cycloundecen** | **CDAN** | **CDEN** | **CDON** | **CDENON** | **CDOL** | **CDENOL** | **Hochsieder** |
| 79 | 1,4 | 0,5 | 0,1 | 89,9 | 3,6 | 1,4 | 1,1 | 2,0 |

Die Selektivität verwertbarer Produkte betrug 97,6 mol-%.

### Beispiel 4: erfindungsgemäß

Die Reaktion wurde in einer Laboranlage gemäß Beispiel 1 durchgeführt. Es wurden kontinuierlich 65 g/h Feed enthaltend 85,4 Gew% Epoxycyclododecan, 9,3 Gew% CDAN und 5,3 % CDEN in den Kreislauf dosiert. Dies entspricht einer Dosierung von 0,31 mol/h Epoxycyclododecan. Aus der Anlage wurde kontinuierlich Produkt über einen Überlaufrohr ausgetragen, so dass der Füllstand im Vorlagebehälter konstant blieb. Außerdem wurden kontinuierlich 5 NL/h Stickstoff in die Anlage dosiert, was eine Stoffmenge von 0,20 mol/h N₂ entspricht. Somit beträgt das Verhältnis der Stoffmenge Stickstoff zu der Stoffmenge Epoxycyclododecan 0,67. Mit einem Vordruckregler wurde ein Gesamtdruck in der Anlage von 1,2 bar eingestellt und das Abgas wurde kontinuierlich aus der Anlage ausgetragen.

Nach 48 h Laufzeit war das Reaktionsgemisch im stationären Zustand. Es wurde ein Umsatz des Epoxids von 78 % erreicht. Die Selektivität des CDON betrug 89 mol-% und es wurden lediglich 2,7 mol-% Hochsieder gebildet.

Der Anteil von Kohlenstoffmonoxid im Abgas wurde mit IR-Spektrometrie ermittelt und betrug 1,71 mol-%. Bei einem Gesamtdruck von 1,2 bar wurde ein Partialdruck von Kohlenstoffmonoxid von 21 mbar erreicht.

**Tabelle 4. Umsatz des Epoxids (mol-%, GC mit externem Standard) und Selektivität der verschiedenen Produkte (mol-%, GC mit externem Standard)**

| **Umsatz Epoxid (mol-%)** | **Selektivität (mol-%)** | | | | | | | |
|---|---|---|---|---|---|---|---|---|
| | **Cycloundecan + Cycloundecen** | **CDAN** | **CDEN** | **CDON** | **CDENON** | **CDOL** | **CDENOL** | **Hochsieder** |
| 77 | 1,5 | 0,8 | 0,1 | 88,5 | 5,2 | 0,1 | 1,0 | 2,9 |

Die Selektivität verwertbarer Produkte betrug 95,6 mol-%.

### Beispiel 5: erfindungsgemäß

Die Reaktion wurde in einer Laboranlage gemäß Beispiel 1 durchgeführt. Es wurden kontinuierlich 65 g/h Feed enthaltend 85,4 Gew% Epoxycyclododecan, 9,3 Gew% CDAN und 5,3 % CDEN in den Kreislauf dosiert. Es entspricht eine Dosierung von 0,31 mol/h Epoxycyclododecan. Aus der Anlage wurde kontinuierlich Produkt über einen Überlaufrohr ausgetragen, so dass der Füllstand im Vorlagebehälter konstant blieb. Außerdem wurde kontinuierlich 20 NL/h Stickstoff in die Anlage dosiert, was eine Stoffmenge von 0,81 mol/h N₂ entspricht. Somit beträgt das Verhältnis der Stoffmenge Stickstoff zu der Stoffmenge Epoxycyclododecan 2,6. Mit einem Vordruckregler wurde ein Gesamtdruck in der Anlage von 1,2 bar eingestellt und das Abgas wurde kontinuierlich aus der Anlage ausgetragen.

Nach 48 h Laufzeit war das Reaktionsgemisch im stationären Zustand. Es wurde ein Umsatz des Epoxids von 76 % erreicht. Die Selektivität des CDON betrug 90 mol-% und es wurden lediglich 2,3 mol-% Hochsieder gebildet.

Der Anteil von Kohlenstoffmonoxid im Abgas wurde mit IR-Spektrometrie ermittelt und betrug 0,70 mol-%. Bei einem Gesamtdruck von 1,2 bar wurde ein Partialdruck von Kohlenstoffmonoxid von 8 mbar erreicht.

**Tabelle 5. Umsatz des Epoxids (mol-%, GC mit externem Standard) und Selektivität der verschiedenen Produkte (mol-%, GC mit externem Standard)**

| **Umsatz Epoxid (mol-%)** | **Selektivität (mol-%)** | | | | | | | |
|---|---|---|---|---|---|---|---|---|
| | **Cyclo-undecan + Cycloundecen** | **CDAN** | **CDEN** | **CDON** | **CDENON** | **CDOL** | **CDENOL** | **Hochsieder** |
| 76 | 1,6 | 0,5 | 0,1 | 90,2 | 3,6 | 1,0 | 0,7 | 2,3 |

Die Selektivität verwertbarer Produkte betrug 96,1 mol-%.

### Ergebnis

Mit dem Beispielen 1 bis 5 konnte demonstriert werden, dass eine Verringerung des CO-Partialdrucks mittels eines inerten Gasgemisches auf unter 50 mbar der Anteil an Hochsiedern als Nebenprodukt aus der Reaktion des Epoxids zum Keton wesentlich verringert.

**Tabelle 6. Übersicht über die Partialdrücke und die daraus resultieren Anteile der Reaktionsprodukte**

| **Beispiel** | **Partialdruck (CO) in mbar** | **Gesamtdruck im Reaktor in bar** | **Selektivität in mol-%** | **Selektivität mol-%** |
|---|---|---|---|---|
| | | | **CDON** | **Hochsieder** |
| 1 | 83 | 3,2 | 6,8 | 17 |
| 2 | 59 | 3,2 | 37,4 | 16,5 |
| 3* | 27 | 3,2 | 89,9 | 2 |
| 4* | 21 | 1,2 | 88,5 | 2,9 |
| 5* | 8 | 1,2 | 90,2 | 2,3 |

| | | | | |
|---|---|---|---|---|
| * erfindungsgemäß | | | | |

## Patentansprüche

1. Verfahren zur kontinuierlichen Herstellung von Ketonen aus einer mindestens eine Epoxidgruppe enthaltenden Verbindung in einer Vorrichtung (6) mindestens umfassend mindestens einen Festbettreaktor (3), wobei der mindestens eine Festbettreaktor (3) eine Katalysatorzusammensetzung enthält, welche mindestens ein Edelmetall und mindestens ein Metalloxid umfasst, **dadurch gekennzeichnet, dass**
a. der Druck im Reaktor (3) vor dem Katalysator mindestens 1,2 bar beträgt,
b. die Gasphase des Reaktors maximal 0,2 bar Wasserstoff-Partialdruck aufweist, vorzugsweise keinen Wasserstoff, und
c. nach dem Katalysator in der Gasphase des Reaktors ein Kohlenstoffmonoxid-Partialdruck von 50 mbar oder weniger durch Einleiten mindestens eines Inertgases eingestellt ist.

2. Verfahren nach Anspruch 1, **dadurch gekennzeichnet, dass** in der Gasphase des Festbettreaktors ein Kohlenstoffmonoxid-Partialdruck von 30 mbar oder weniger eingestellt ist.

3. Verfahren nach einem der vorherigen Ansprüche, **dadurch gekennzeichnet, dass** das Metalloxid des Katalysatorsystems Titandioxid, Zirconiumdioxid oder Mischungen daraus enthält oder aus mindestens einem der zuvor genannten Oxide besteht.

4. Verfahren nach einem der vorherigen Ansprüche, **dadurch gekennzeichnet, dass** der Gesamtdruck im Reaktor 4 bar oder weniger beträgt, bevorzugt 2 bar oder weniger.

5. Verfahren nach einem der vorherigen Ansprüche, **dadurch gekennzeichnet, dass** das Verhältnis der Stoffmenge an Inertgas und der Stoffmenge der Epoxidgruppe enthaltenden Verbindung mindestens 0,5 beträgt.

6. Verfahren nach einem der vorherigen Ansprüche, **dadurch gekennzeichnet, dass** dem Festbettreaktor mindestens ein weiterer Festbettreaktor nachgeschaltet ist.

7. Verfahren nach Anspruch 6, **dadurch gekennzeichnet, dass** eine Gaseinleitung des Abgases zwischen den Festbettreaktoren im Gegenstrom erfolgt.

8. Verfahren nach einem der vorherigen Ansprüche, **dadurch gekennzeichnet, dass** die mindestens eine Epoxidgruppe enthaltenden Verbindung eine cycloaliphatische Verbindung mit 4 bis 20 C-Atomen ist.

9. Verfahren nach Anspruch 7, **dadurch gekennzeichnet, dass** die mindestens eine Epoxidgruppe enthaltenden Verbindung Monoepoxy-cyclododecan umfasst.

10. Verfahren zur Synthese von Lactamen, umfassend die Schritte
a. Umlagerung einer mindestens eine Epoxidgruppe enthaltenden Verbindung zum Keton,
b. Oximierung des Ketons zum Oxim
c. Beckmann-Umlagerung des Oxims zum Lactam,
**dadurch gekennzeichnet, dass** die Umlagerung a. nach einem der vorherigen Ansprüche durchgeführt wird.

## Claims

1. Method for the continuous production of ketones from a compound comprising at least one epoxide group in a device (6) at least comprising at least one fixed bed reactor (3), wherein the at least one fixed bed reactor (3) comprises a catalyst composition comprising at least one noble metal and at least one metal oxide, **characterized in that**
a. the pressure in the reactor (3) upstream of the catalyst is at least 1.2 bar
b. the gas phase of the reactor has a hydrogen partial pressure of at most 0.2 bar, preferably no hydrogen, and
c. in the gas phase of the reactor downstream of the catalyst a carbon monoxide partial pressure of 50 mbar or less is set by introducing at least one inert gas.

2. Method according to Claim 1, **characterized in that** a carbon monoxide partial pressure of 30 mbar or less is set in the gas phase of the fixed bed reactor.

3. Method according to either of the preceding claims, **characterized in that** the metal oxide of the catalyst system comprises titanium dioxide, zirconium dioxide or mixtures thereof or at least one of the oxides specified above.

4. Method according to any of the preceding claims, **characterized in that** the total pressure in the reactor is 4 bar or less, preferably 2 bar or less.

5. Method according to any of the preceding claims, **characterized in that** the ratio of the amount of inert gas and the amount of the epoxide group-containing compound is at least 0.5.

6. Method according to any of the preceding claims, **characterized in that** at least one further fixed bed reactor is downstream of the fixed bed reactor.

7. Method according to Claim 6, **characterized in that** offgas is introduced in countercurrent between the fixed bed reactors.

8. Method according to any of the preceding claims, **characterized in that** the compound comprising at least one epoxide group is a cycloaliphatic compound having 4 to 20 carbon atoms.

9. Method according to Claim 7, **characterized in that** the compound comprising at least one epoxide group comprises monoepoxycyclododecane.

10. Method for the synthesis of lactams comprising the steps of
a. rearrangement of a compound comprising at least one epoxide group to the ketone,
b. oximation of the ketone to give the oxime,
c. Beckmann rearrangement of the oxime to give the lactam,
**characterized in that** the rearrangement a. is carried out according to any of the preceding claims.

## Revendications

1. Procédé pour la fabrication continue de cétones à partir d'un composé contenant au moins un groupe époxyde dans un dispositif (6) au moins comprenant au moins un réacteur à lit fixe (3), ledit au moins un réacteur à lit fixe (3) contenant une composition de catalyseur, qui comprend au moins un métal noble et au moins un oxyde métallique, **caractérisé en ce que**
a. la pression dans le réacteur (3) avant le catalyseur est d'au moins 1,2 bar,
b. la phase gazeuse du réacteur présente au plus 0,2 bar de pression partielle d'hydrogène, de préférence pas d'hydrogène, et
c. après le catalyseur, dans la phase gazeuse du réacteur, une pression partielle de monoxyde de carbone de 50 mbar ou moins est ajustée par introduction d'au moins un gaz inerte.

2. Procédé selon la revendication 1, **caractérisé en ce qu'**une pression partielle de monoxyde de carbone de 30 mbar ou moins est ajustée dans la phase gazeuse du réacteur à lit fixe.

3. Procédé selon l'une quelconque des revendications précédentes, **caractérisé en ce que** l'oxyde métallique du système de catalyseur contient du dioxyde de titane, du dioxyde de zirconium ou des mélanges de ceux-ci ou est constitué par au moins un des oxydes susmentionnés.

4. Procédé selon l'une quelconque des revendications précédentes, **caractérisé en ce que** la pression totale dans le réacteur est de 4 bar ou moins, de préférence de 2 bar ou moins.

5. Procédé selon l'une quelconque des revendications précédentes, **caractérisé en ce que** le rapport entre la quantité de matière de gaz inerte et la quantité de matière du composé contenant un groupe époxyde est d'au moins 0,5.

6. Procédé selon l'une quelconque des revendications précédentes, **caractérisé en ce qu'**au moins un réacteur à lit fixe supplémentaire est raccordé en aval du réacteur à lit fixe.

7. Procédé selon la revendication 6, **caractérisé en ce qu'**une introduction de gaz du gaz d'échappement a lieu à contre-courant entre les réacteurs à lit fixe.

8. Procédé selon l'une quelconque des revendications précédentes, **caractérisé en ce que** le composé contenant au moins un groupe époxyde est un composé cycloaliphatique de 4 à 20 atomes C.

9. Procédé selon la revendication 7, **caractérisé en ce que** le composé contenant au moins un groupe époxyde comprend du monoépoxy-cyclododécane.

10. Procédé de synthèse de lactames, comprenant les étapes suivantes :
a. le réarrangement d'un composé contenant au moins un groupe époxyde en cétone,
b. l'oximation de la cétone en oxime,
c. le réarrangement de Beckmann de l'oxime en lactame,
**caractérisé en ce que** le réarrangement a. est réalisé selon l'une quelconque des revendications précédentes.
